# EUROPEAN PATENT SPECIFICATION

(11) **EP 1 238 116 B1**
(45) Date of publication and mention of the grant of the patent: **16.04.2008**
(21) Application number: 99961234.4
(22) Date of filing: 17.12.1999
(51) Int. Cl.: C12Q 1/68

(54) **PROCESS FOR LABELING A NUCLEIC ACID**
VERFAHREN ZUR MARKIERUNG VON NUKLEINSÄUREN
PROCEDE DE MARQUAGE D'UN ACIDE NUCLEIQUE

(43) Date of publication of application: 11.09.2002
(73) Proprietor: BIO MERIEUX, 69280 Marcy l'Etoile (FR); GEN-PROBE INCORPORATED, San Diego, CA 92121-4362 (US)
(72) Inventor: BANERJEE, Aloke, Raj, San Diego, CA 92126 (US); BECKER, Michael, M., San Diego, CA 92131 (US); BROWNE, Kenneth, A., Poway CA 92064 (US); FRIEDENBERG, Matthew, C., San Diego, CA 92131 (US); HAJJAR, Fred F., Oceanside, CA 92056 (US); LAAYOUN, Ali, 69007 Lyon (FR); MENOU, Lionel, F-69005 Lyon (FR); TORA, Christelle, 38780 Oytier Saint Oblas (FR)
(74) Representative: Guerre, Dominique
(86) International application number: PCT/IB1999/002073
(87) International publication number: WO 2001/044507

(56) References cited:
- EP-A- 0 801 072
- WO-A-93/20241
- WO-A-98/27229
- WO-A-99/65926
- DE-A- 19 815 864
- US-A- 5 171 853
- MAG, M. ET AL.: "Synthesis and selective cleavage of an oligodeoxynucleotide containing a bridged internucleotide 5'-phosphorothioate linkage" NUCLEIC ACIDS RES, vol. 19, 1991, pages 1437-41, XP000857921

## Description

### Field of the Invention

The present invention relates to a novel process for labeling a nucleic acid, and particularly relates to a chemical method for simultaneously fragmenting and labeling nucleic acids.

### Background

There are a large number of methods for labeling nucleotides, oligonucleotides or nucleic acids (herein referred to by the term polynucleotides). Polynucleotides can be labeled either during synthesis (e.g., by incorporating at least one labeled nucleotide) or by adding a label to the polynucleotide after synthesis. For example, one method attaches the label to the base, whether the latter is a natural base or a modified base. A second method attaches the label to the sugar, again whether it is a natural sugar or a modified sugar. A third method attaches the label to the phosphate. Often, preferred methods attach the label to the base or to the sugar, because such methods are more convenient and provide more options for labeling. See, for example, the methods disclosed in EP-A-0.329.198, EP-A-0.302.175, EP-A-0.097.373, EP-A-0.063.879, US-A-5,449,767, US-A-5,328,824, WO-A-93/16094, DE-A-3.910.151 and EP-A-0.567.841 in the case of base labeling, or EP-A-0.286.898 in the case of sugar labeling. Attaching the label to the phosphate is more complex because nucleic acids are water soluble and the reactivity of the phosphate in an aqueous solution is low. Nonetheless, phosphate labeling methods have been described in EP-A-0.280.058. In this method, the label is attached to the phosphate, which is attached to the sugar in the 3' and/or 5' positions, for a deoxyribonucleotide, and in the 2', 3' and/or 5' positions for a ribonucleotide. The labeled nucleotide may be incorporated into the polynucleotide or oligonucleotide during synthesis.

However, the labeling described in EP-A-0.280.058 does not uniformly label the nucleic acids. The incorporation of the labeled nucleotides into the polynucleotides cannot be controlled and depends on the composition of synthesized polynucleotides. Thus, some polynucleotides may contain a large number of labeled nucleotides whereas others may not contain any. As a result, the intensity of the signal emitted by these labeled nucleic acids will not be uniform, making it difficult to interpret the results when detecting the nucleic acids.

Another method, described in US-A-5,317,098 relates to nucleic acids (e.g., 15-mers) which are labeled at their 5' ends by using imidazole and a linker arm. Furthermore, phosphate is added to nucleic acids by using a kinase, thus adding at least one additional step. When this method is used to label larger nucleic acids, the specific activity is low because this technique labels only the 5' end.

In some instances, fragmentation of a labeled nucleic acid is also desirable, such as to increase hybridization kinetics of the labeled fragment with another nucleic acid by decreasing the size of the labeled polynucleotide, In contrast, hybridization using a larger labeled polynucleotide may result in a quantitative and qualitative loss of the signal. Fragmentation of a labeled polynucleotide may also be needed to reduce steric hindrance.

Steric hindrance may result from the length of the nucleic acid and the existence of secondary structures. Fragmentation helps to remove these structures and, thus, optimize hybridization. Steric hindrance plays a particularly important role in hybridization to surfaces which contain a high density of capture probes, for example, in high-density arrays of probes as occur on "DNA chips" (GENECHIP®; Affymetrix, Santa Clara, CA, USA; (Chee et al., 1996, Science 274:610-614; Caviani Pease et al., 1994, Proc. Natl. Acad. Sci. USA 91:5022-5026 ; US 5,445,934 ; US 5,744,305; Ramsay, 1998, Nature Biotechnol. 16:40-44; Ginot, 1997, Human Mutation 10:1-10; Cheng et al., 1996, Molec. Diagnosis 1(3):183-200; Livache et al., 1994, Nucl. Acids Res. 22(15): 2915-2921; Cheng et al., 1998, Nature Biotechnol. 16: 541-546; US 5,525,464, US 5,202,231, US 5,807,522 and US 5,700,637).

Methods for fragmenting nucleic acids are known in the art. For example, fragmentation can be enzymatic (i.e. by nucleases such as DNases or RNases). This generates small fragments having 3'-OH, 5'-OH, 3'-phosphate and 5'-phosphate ends. Alternatively, fragmentation can be chemical. For example, for DNA, it is possible to depurinate or depyrimidinate the DNA, which are then fragmented in the presence of a base (i.e., "β-climination"). DNA can be fragmented by oxidation, alkylation or free radical addition mechanisms. Metal cations, which arc often combined with organic molecules which may function as chemical catalysts, for example imidazole, are used for fragmenting RNA. This fragmentation is preferably carried out in an alkaline medium and generates fragments having 3'-phosphate ends. Nucleic acid fragmentations, using silver or mercuric ions under mild conditions have been described (Mag et al., 1991, Nucleic Acid Research, 19(7), 1437-1441), targeting specific bridged 5'-phosphorothioate linkages of modified oligo(deoxy)nucleotides.

Different nucleic acid fragmentation techniques have been described in Trawick et al., 1998, Chem Rev. 98: 939-960 and Oivanen et al., 1998, Chem Rev. 98: 961-990.

A method for fragmenting and labeling RNA is described in WO-A-88/04300, in which the fragmentation is carried out using RNA which possesses enzymatic properties (ribozymes). Fragmentation by ribozymes releases a nucleic acid (5') HO end and a nucleic acid (3') HO-PO₂ end. Radioactive labeling is then effected by incorporating a radioactive phosphate, derived from GTP, at the 5'OH end; no phosphate resulting from fragmentation is used in labeling. Fragmentation carried out by ribozymes implies specificity between the ribozymes and the target nucleic acids to be cleaved, after which the phosphate acts as the label. Selective fragmentation of RNA containing at least on G-U wobble base pair by photo oxidation reaction in presence of divalent metal action have been disclosed (EP 0 801 072 A2).

Reliable diagnostic tests based on nucleic acid amplification techniques often include steps to control contamination by nucleic acids that can otherwise serve as targets for further amplification. Several decontamination procedures have been developed (Longo et al., 1990, Gen. 93: 125-128; Abravaya et al., in Nucleic Acid Amplification Technologies, p 125-133; (1997) Eds. Lee et al. (Eston Publishing, 1997) at pp. 125-133; EP O 709 468 A1 and US Patent No. 5,605,796). These procedures make the amplified nucleic acid product incapable of being a target for further amplification, generally by degrading nucleic acids that would otherwise serve as targets (e.g., by using irradiation, endonucleases, uracil DNA glycosylase, primer modification or photochemical methods). Some of these methods are difficult to implement, are inefficient or introduce additional steps and/or toxic compounds into a procedure (e.g., UV inactivation, photochemical degradation, primer modification). Enyzmatic methods use enzymes that are often expensive and incompatible with amplification and/or detection buffers. Thus, there remains a need for efficient and convenient methods of target nucleic acid removal.

### Summary of the Invention

One aspect of the present invention is a process of fragmenting and labeling a synthetic or natural nucleic acid, comprising the steps of providing a mixture containing a nucleic acid, a labeling agent containing a detectable label a chemical catalyst, and at least one multivalent metal cation in a substantially aqueous solution; chemically fragmenting the nucleic acid in the mixture to produce a multiplicity of nucleic acid, fragments; and attaching at least one label to at least one of the nucleic add fragments to produce a detectably labeled nucleic acid fragment In one embodiment of the process the nucleic acid is DNA, RNA, a chimeric DNA-RNA polymer, DNA comprising at least one thiophosphate nucleotide or RNA comprising at least one thiophosphate nucleotide. In another embodiment, reagents used in the fragmenting and attaching steps are added to an in vitro nucleic acid amplification mixture. According to yet another embodiment, the at least one label is attached to at least one phosphate of the nucleic acid fragments. The process may further comprise the step of treating the mixture after the fragmenting and attaching steps to decrease or eliminate unattached labeling agent. In one embodiment, the treating step consists in adding a quencher to the mixture after the fragmenting and attaching steps. Preferred quenchers include a pyrophosphate, thiol derivative, chelating agent, phosphate anion or carbonate anion. In another embodiment, the treating step physically separates the labeled nucleic acid fragment from unattached labeling agent in the mixture after the fragmenting and attaching steps. The treating step may further include adding an acid to the mixture after the fragmenting and attaching steps. Another embodiment of the treating step further includes adding a chelating agent to the mixture after the fragmenting and attaching steps. In one embodiment, the treating step uses an organic solvent to separate the labeled nucleic acid fragment from the unattached labeling agent. Preferred organic solvents are 1-butanol, 2-butanol, isopentyl alcohol, 1-pentanol or cyclohexanol. In another embodiment, the treating step separates the labeled nucleic acid fragment from the unattached labeling agent by using solid phase extraction of the nucleic acid fragments on a solid support. Preferably, the solid support is beads, gels, ion exchange resin, reverse phase resin, silica matrix or a membrane. In another embodiment, the labeled nucleic acid fragment is eluted from the solid support by using a buffer containing betaine. One embodiment includes a treating step that precipitates the labeled nucleic acid fragments at ambient temperature from a solution that contains betaine, DTAB and unlabeled nucleic acid. Another embodiment uses a treating step that dilutes an *in vitro* nucleic acid amplification mixture. In one embodiment, the fragmenting and attaching steps are performed in a single reaction mixture, whereas in another embodiment, the fragmenting and attaching steps are effected in separate steps. In preferred embodiments, the attaching step attaches a label to an internal or terminal thiophosphate or to an internal or terminal phosphate. Preferably, the chemical catalyst is a general base selected from the group consisting of imidazole, a substituted analogue of imidazole, and a compound that includes an imidazole ring or substituted analogue of an imidazole ring. Preferred chemical catalysts are selected from the group consisting of N-methylimidazole, MOPS, HEPES, PIPES, and bioorganic polyamines. According to the process, the nucleic acid is an RNA or RNA comprising at least one thiophosphate nucleotide, and the multivalent metal cation is Sr²⁺, Ba²⁺, Pb²⁺, Cd²⁺, Fe²⁺, Ni²⁺, Ru³⁺, Ce³⁺, Eu³⁺, Tb³⁺, Tm³⁺, Yb³⁺ or Lu³⁺, or the nucleic acid is DNA or DNA comprising at least one thiophosphate nucleotide, and the multivalent metal cation is Mn²⁺, Zn²⁺, Be²⁺, Cr³⁺, Pb²⁺, In³⁺, Tb³⁺, Ce³⁺, Yb³⁻ or Ni²⁺. Preferred embodiments of the process use a multivalent metal cation that is Mn²⁺, Zn²⁺, Tb³⁺ or Ce³⁺. In preferred embodiments, the mixture contains the labeling agent in a concentration of between 0.1 mM to 4 mM, more preferably between 0.1 mM to 1 mM. In preferred embodiments, the labeling agent is between 0.3 mM to 0.55 mM. Preferably, the mixture contains a labeling agent that contains alkyl halide or haloacetamide reactive functions. Preferred labeling agents are 5-(bromomethyl)fluorescein, 6-(bromomethyl)fluorescein, 6-iodoacetamidofluorescein or 5-iodoacetamidofluorescein.

### Detailed Description of the Invention

The present invention includes methods to chemically fragment nucleic acids and simultaneously label the fragments with a detectable label, such as a fluorescent compound. The labeled fragments can then be detected by a variety of methods. This process is useful for preparing labeled nucleic acids, such as fragments to be bound to immobilized probes or detection probes. This process can limit nonspecific signals that result from the labeling step, particularly when combined with nucleic acid purification steps using any of a variety of methods. Furthermore, this process provides nucleic acid fragments that are relatively uniformly labeled. The fragmentation process results in fragments that are of an optimum size for hybridization to nucleic acid probes used in detection of the fragmented nucleic acids, thus making the detecting step more rapid and efficient.

The present invention relates to a process for labeling a synthetic or natural nucleic acid, characterized by the steps of fragmenting a nucleic acid by chemical processes and attaching a label to the fragmented nucleic acid. The process may optionally include treating the labeling mixture to decrease the amount of labeling agent therein.

By "nucleic acid" is meant DNA, RNA or chimeric DNA-RNA polymers (single-stranded, double-stranded or partially double-stranded), and nucleic acid molecules made partially or completely of nucleotide analogues or abasic residues that may be present in the sequence. The DNA or RNA may be purified from a natural source (e.g., extracted from a cell) or be synthetically prepared (e.g., by chemical, enzymatic or other known synthesis methods). In some embodiments, the nucleic acid is amplified DNA, amplified RNA, or a mixture thereof, which may further include at least one thiophosphate nucleotide. A phosphate may be a terminal phosphate which is located at the 3' end and/or the 5' end of the nucleic acid fragments, an internal phosphate or an internal thiophosphate.

The term nucleic acid includes conventional RNA and DNA, as well as analogs thereof. The "backbone" of a nucleic acid may be made up of a variety of linkages known in the art, including one or more of sugar-phosphodiester linkages, peptide-nucleic acid bonds (sometimes referred to as "peptide nucleic acids" as described by Hydig-Hielsen et al., PCT Pat. App. No. WO 95/32305), phosphorothioate linkages, methylphosphonate linkages or combinations thereof. Sugar moieties of the nucleic acid may be either ribose or deoxyribose, or similar compounds having known substitutions, such as, for example, 2' methoxy substitutions and 2' halide substitutions (e.g., 2'-F). The nitrogenous bases may be conventional bases (A, G, C, T, U), known analogs thereof (e.g., inosine or "I"; see The Biochemistry of the Nucleic Acids 5-36, Adams et al., ed., 11th ed., 1992), known derivatives of purine or pyrimidine bases (*e.g*., N⁴-methyl deoxygaunosine, deaza- or aza-purines and deaza- or aza-pyrimidines, pyrimidine bases having substituent groups at the 5 or 6 position, purine bases having an altered or a replacement substituent at the 2, 6 or 8 positions, 2-amino-6-methylaminopurine, O⁶-methylguanine, 4-thio-pyrimidines, 4-amino-pyrimidines, 4-dimethylhydrazine-pyrimidines, and O⁴-alkyl-pyrimidines; *see*, Cook, PCT Pat. App. No. WO 93/13121) and "abasic" residues where the backbone includes no nitrogenous base for one or more residues of the polymer (U.S. Pat. No. 5,585,481). A nucleic acid may comprise only conventional sugars, bases and linkages found in RNA and DNA, or may include both conventional components and substitutions (e.g., conventional bases linked via a methoxy backbone, or a nucleic acid including conventional bases and one or more base analogs).

By "amplification" is meant any known procedure for obtaining multiple copies of a target nucleic acid sequence or its complement or fragments thereof. Known amplification methods include, for example, transcription-mediated amplification, replicase-mediated amplification, polymerase chain reaction (PCR) amplification, ligase chain reaction (LCR) amplification and strand-displacement amplification (SDA). Replicase-mediated amplification uses self-replicating RNA molecules, and a replicase such as QB-replicase (U.S. Pat. No. 4,786,600; PCT Pat. App. No. WO 90/14439). PCR amplification is well known and uses DNA polymerase, primers and thermal cycling to synthesize multiple copies of the two complementary strands of DNA or cDNA (*e.g., see* U.S. Pat. Nos. 4,683,195, 4,683,203, and 4,800,159; Methods in Enzymology, 1987, Vol. 155: 335-350). LCR amplification uses at least four separate oligonucleotides to amplify a target and its complementary strand by using multiple cycles of hybridization, ligation, and denaturation (EP Pat. App. Pub. No. 0 320 308). SDA is a method in which a primer contains a recognition site for a restriction endonuclease such that the endonuclease will nick one strand of a hemimodified DNA duplex that includes the target sequence, followed by amplification in a series of primer extension and strand displacement steps (Walker et al., 1992, Proc. Natl. Acad. Sci. USA 89:392-396; and U.S. Pat. No. 5,422,252). Transcription-associated amplification is a preferred embodiment of the present invention. It will, however, be apparent to one skilled in the art that the methods of the present invention can be readily used with nucleic acid amplified by any method.

By "transcription-mediated amplification" or "transcription-associated amplification" is meant any type of nucleic acid amplification that uses an RNA polymerase to produce multiple RNA transcripts from a nucleic acid template (see U.S. Pat. Nos. 4,868,105 and 5,124,246, 5,130,238, 5,399,491 and 5,554,516, 5,437,990; and PCT Application Nos. WO 93/22461, WO 88/01302 and WO 88/10315, WO 94/03472 and WO 95/03430. Transcription-associated amplification generally employs an RNA polymerase, a DNA polymerase, deoxyribonucleoside triphosphates, ribonucleoside triphosphates, and a promoter-template complementary oligonucleotide, and optionally may include one or more analogous oligonucleotides. Preferred methods of transcription-mediated amplification (TMA) are disclosed in detail in U.S. Pat. Nos. 5,399,491 and 5,554,516 and PCT Application Nos. WO 93/22461, WO 94/03472 and WO 95/03430.

Chemical fragmentation of the nucleic acid is carried out by using at least one multivalent metal cation, which is combined with a chemical catalyst. Chemical catalysts used in the fragmentation process are those that act as a general base, including, for example, imidazole, a substituted analogue (e.g., N-methylimidazole), or a chemical compound that includes an imidazole ring or a substituted analogue thereof. Additional chemical catalysts that may be used for nucleic acid fragmentation include MOPS, HEPES, PIPES, and bioorganic polyamines, such as spermine, spermidine and putrescine (Arkadiusz et al., 1999, Nucleic Acids Res. 27: 3931-3937).

In preferred methods of the present invention, the nucleic acid to be fragmented is RNA and the multivalent metal cation is at least one of Sr²⁺, Ba²⁺, Pb²⁺, Cd²⁺, Fe²⁺, Ni²⁺, Ru³⁺, Ce³⁺, Eu³⁺, Lu³⁺, Tb³⁺, Tm³⁺ or Yb³⁺. In other embodiments in which RNA is fragmented, the preferred multivalent metal cation is at least one of Eu²⁺, Pb²⁺, Ce³⁺, Cr³⁺, Tb³⁺ or Yb³⁺.

In preferred methods of the present invention, the nucleic acid to be fragmented is DNA and the multivalent metal cation is at least one of Mn2⁺, Zn²⁺, Be²⁺, Pb²⁺, Ni²⁺, Cr³⁺, Ce³⁺, In3⁺, Tb³⁺ or Yb³⁺. In another preferred embodiment, the nucleic acid to be fragmented is DNA that includes at least one thiophosphate nucleotide, and the metal cation is Tb³⁺.

Additional chemical fragmentation of DNA that optionally contains at least one thiophosphate nucleotide may be effected by using metal chelating agents (Sentagne et al., 1992, J. Photochem. Photobiol. B. 16: 47-59), photoactivatable compounds (Nadji, 1992, Am. Chem. Soc. 112: 9266-9269) and alkylating agents (Povsic et al., 1990, J. Am. Chem. Soc. 112: 9428-9430).

By "labeling" is meant attachment of a detectable label to a nucleic acid to generate a detectable signal associated with the nucleic acid. The compound which comprises the label is the labeling agent. Known labels include enzymes (e.g., alkaline phosphatase) that produce a signal e.g., by colorimetry, fluorescence, luminescence; chromophores (e.g, fluorescent and luminescent compounds and dyes); electron dense groups that are detectable by electron microscopy or by measuring electrical properties; size-dependent detectable groups that can be detected using optical or physical methods; and radionuclides.

Labeling agents of the present invention include compounds that include alkyl halide (e.g., bromoalkyl or bromomethyl) and haloacetamide reactive functions (e.g., iodoacetimido group). Such labeling agents include, for example, 5-(bromomethyl)fluorescein, 6-(bromomethyl)fluorescein, 6-iodoacetamidofluorescein and 5-iodoacetamidofluorescein, Those skilled in the art will appreciate that other reactive compounds may equivlantly be used based on their known chemical reactivity, such as, for example, hydrazine, alkoxylamine, alkyl or aryl halides and maleimide. Preferred concentrations of the labeling agent are in the range having a lower limit of 0.01 mM and an upper limit of 10 mM, more preferably in a range having a lower limit of 0.1 mM and an upper limit of 4 mM. In some preferred embodiments, the labeling agent is used in a concentration range of between 0.1 mM to 1 mM, and in other embodiments in a concentration range of between 0.3 mM to 0.55 mM. When the labeling agent is 5-(bromomethyl)fluorescein, the fragmentation and labeling steps preferably occur in the presence of Mn²⁺ at 15 mM to 60 mM, imidazole in a range between 5 mM to 30 mM and at pH in a range between 6.8 and 7.2. In one embodiment of the present invention, nucleic acid fragmentation and labeling are effected in one reaction mixture, additionally providing a method for target inactivation, eliminating the need for a post-detection step of removing any remaining target nucleic acid. For example, an RNA target molecule present in the fragmentation and labeling reaction mixture would be degraded into short fragments.

An advantage of the present invention is that nucleic acid fragmentation and labeling reaction also serves as a decontamination tool. That is, the process fragments RNA molecules present in the amplification mixture thus removing potential targets for further amplification from the system because the fragmented RNA fragments are incapable of being a target for further amplification.

In another embodiment of the present invention fragmentation and labeling are effected in two steps, using either the entire volume of amplification reaction or a portion thereof.

In another embodiment of the invention, a treating step is included after the fragmentation and labeling steps to decrease or eliminate unreacted labeling agent. This step limits or eliminates non-specific signals that otherwise result from the presence of the unreacted label. Such a treating step may involve adding a quencher compound to the labeling reaction, or may involve physically separating the labeled nucleic acid fragments from unreacted labeling agents using any of a variety of methods.

A quencher is a compound that (1) forms soluble complexes with the metal cations in the reaction mixture, (2) precipitates the metal cations from the reaction mixture or (3) reacts with the residual labeling agent, thus, effectively removing it from the mixture. Soluble complexes can be formed, for example by using a chelating agent such as EDTA. Precipitable complexes can be formed by adding pyrophosphate anions. Quencher compounds can readily be selected by one skilled in the art based on standard chemical interactions with the particular reactive groups involved in the labeling reaction. Preferred quenchers include pyrophosphate or thiol derivatives such as dithiothreitol, cysteine, gluthatione, mercaptosuccinic acid. Alternatively, or in addition to using a quencher, the treating step may remove unreacted label by methods that physically separate the labeled nucleic acid from the unattached labeling agent. Separating the unattached label from the labeled nucleic acid fragments may involve extracting the unreacted label into at least one organic solvent, such as 1-butanol, 2-butanol, isopentyl alcohol, 1-pentanol or cyclohexanol. A preferred solvent is 1-butanol. Another preferred extraction method includes acidifying the labeleing mixture before the solvent extraction. Alternatively, the organic solvent used for extraction is mixed with ethylene-diamine-tetraacetic acid (EDTA) before the extraction process begins.

Accordingly to another embodiment, the treating step is not carried out by the precipitation of nucleic acid fragments in a mixture of sodium acetate and cold isopropanol.

Purification of labeled nucleic acid fragments may also be effected by removing the unattached label using a solid phase extraction method. The solid phase support for such an extraction method is preferably beads, gel-filtration resins (e.g., Sephadex™, Sephacryl™ and BioGel™), gels or membranes (e.g., nylon, nitrocellulose, glass fiber or silica). Particularly preferred solid phase extraction media include gel-filtration resin Sephadex™ G-50, silica membranes and silica beads. The solid phase extraction methods are preferably performed using a column to contain the solid phase support and the solutions can be moved through the column using gravity-flow, vacuum suction, or positive pressure such as by centrifugation or used of a syringe attached to the top of the column. Before applying the labeling reaction mixture to a solid phase medium for extraction, a chelating agent such as EDTA is added to the mixture. In one preferred embodiment, the solid phase is paramagnetic particles coated with silica and the captured labeled nucleic acid fragments are eluted with betaine.

Solid phase purification methods are fast, simple, efficient and does not use organic solvents. Furthermore, due to its binding capacity limit, the solid support can be adapted to remove excess labeled fragments that may cause signal saturation during the detection step.

Additional or alternative methods for treating the labeling mixture to decrease the amount of unreacted labeling agent include precipitating the fragmented nucleic acids in a saline buffer containing betaine, a trialkyl ammonium salt derivative such as DTAB (dodecyl trimethylammonium bromide) or CTAB (cetyl trimethylammonium bromide) and exogeneous DNA. The labeling mixture can also be diluted to lower the concentration of unreacted labeling agent before the detecting step.

Although not wishing to be bound to any particular mechanism, the present methods of fragmenting and labeling nucleic acids may attach the label to a phosphate group or thiophosphate group in the nucleic acid. Such attachment may occur at a terminal or internal phosphate or thiophosphate group in the polymer.

These methods are illustrated by the examples that follow that demonstrate some preferred embodiments of the present invention.

### Example 1: Preparation of RNA amplicons

To produce nucleic acids for fragmentation and labeling the following nucleic acid amplification reactions were performed. These reactions used two different sources of nucleic acid as the target, one derived from *Mycobacterium tuberculosis* and the other derived from the human immunodeficiency virus, HIV-1.

### Mycobacterium 16S amplicons

Amplicons of 16S rRNA of *Mycobacterium tuberculosis* were obtained using transcription mediated amplification (TMA) (Kacian et al., U.S. Pat. Nos. 5,399,491 and 5,554,516; Kacian et al., PCT Patent App. No. WO 93/22461; McDonough et al., PCT Pat. App. No. WO 94/03472; and Ryder et al., PCT Pat. App. No. WO 95/03430). Amplification reactions were carried out using 10⁶ copies of rRNA target (*M. tuberculosis* 16S) and the reagents and methods of the Mycobacterium Tuberculosis Direct (MTD) kit (Gen-Probe Incorporated, San Diego, CA, USA). Primers used in TMA are disclosed in an application entitled "Methods and Compositions for Detecting *Mycobacterium* Species Using Nucleic Acid Amplification" which is being filed separately by the applicants on the day previous to the filing date of this application.

Briefly, after mixing the target nucleic acid, the primers and the amplification substrates, the amplification mixture was incubated at 42°C for 1 hr. TMA product was analyzed by electrophoresis on a 6% polyacrylamide gel containing 7M urea and the separated amplicon products were visualized after ethidium bromide staining, to assess the product size and quantity by comparison with an RNA standard on the gel.

### HIV-1 amplicons

HIV amplicons were prepared using a polymerase chain reaction, as described by Kozal et al. (1996, Nature Med. 2 (7): 753-759). Briefly, DNA was extracted from 10⁶ cocultured cells by first treating the cells with lysis buffer (10 mM Tris-HCl, pH 8.3, 2.5 mM MgCl₂, 0.45% Tween™-20, 50 mM KCl , 0.1 mg/ml proteinase K) for 2 hr at 56°C.

A set of nested PCR reactions were used to amplify the HIV 1 DNA. The first reaction generated a 1200-bp amplicon using around 1.0µg of input DNA and the two following primers:
aattaaccctcactaaagggagaCAGAGCCAACAGCCCCACCA (SEQ ID NO:1, in which a T3 RNA polymerase promoter sequence is shown in lowercase), and
taatacgactcactatagggagaTTTCCCCACTAACTTCTGTATGTCATTGACA (SEQ ID NO:2, in which a T7 RNA polymerase promoter sequence is shown in lowercase). The PCR reaction was carried out in a reaction mixture containing 100 mM Tris-HCl, pH 8.3, 500 mM KCl, 1.5 mM MgCl₂, 0.2 mM each dNTPs, 0.2 µM primers (each) and 1.25 units Taq DNA polymerase. The reaction was incubated at 94°C for 30 s, 55°C for 30 s, 72 °C for 2 min for 25 cycles and 72°C for 10 min for the last cycle.

The second reaction produced a 460-bp sequence, internal to the first amplified sequence, by using the SEQ ID NO:1 primer as used in the first PCR and a third primer having the sequence taatacgactcactatagggagaGGGCCA TCCATTCCTGGCTTTAATTT (SEQ ID NO:3). The reaction was incubated at 94°C for 30 s, 63°C for 30 s, 72 °C for 1 min for 30 cycles, and 72°C for 10 min for the last cycle. The PCR products were transcribed by using 20 U of T3 or T7 RNA polymerase in an *in vitro* reaction containing 40 mM Tris Ac₂, pH 8.1, 100 mM KAc, 30 mM MgAc₂, 10 mM DTT and 1.25 mM rNTPs.

The product size and quantity was assessed on a gel as described above.

For labeling reactions, RNA amplicons were used without further purification.

### Example 2: Background Reduction by Using Extraction with Organic Solvents

Imidazole and MnCl₂ were obtained from Sigma-Aldrich Chimie (St Quentin Fallavier, France) and 5-(bromomethyl)fluorescein was purchased from Molecular Probes (Eugene, OR, USA, reference B 1355).

Amplicons of 16S rRNA were prepared as described in Example 1. Each labeling reaction (100 µl) included: RNA molecules (5 µl of TMA reaction mixture), 6 µl imidazole (0.1 M), 6 µl of MnCl₂ (1M) 2 µl of 5-(bromomethyl)fluorescein (50 mM dissolved in DMF) and 81 µl of pure water. The reactions were mixed and incubated at 65°C for 30 min.

Product was hybridized, detected and analyzed on an immobilized probe array on a DNA chip (GENECHIP®) using the manufacturer's protocol (Affymetrix, Santa Clara, CA, USA). This DNA chip is designed for the detection and 4-L tiling of 16S rRNA of *M. tuberculosis* (region 213-415 of M20940 sequence « Genbank », as described in Troesch et al., 1999, J. Clin. Microbiol. 37(1): 49-55). The results, obtained using functions available on GENECHIP® software (Affymetrix), included the following: BC : nucleotide base call (expressed in percentage); S : mean signal intensities for probe array cells (expressed in relative fluorescence unit : RFU); B : mean background intensities (expressed in RFU); and S/B : ratio of signal to background. For this assay, 96.2% base calling was achieved with a mean signal of 5488 RFU and a background of 2420 RFU, to give a S/B ratio of 2.3.

This result shows that useful base calling and signal intensity can be obtained by using only 5% of amplicons generated in a single amplification reaction. The S/B ratio, however, was relatively low.

In other experiments, smaller volumes of fragmentation and labeling mixtures were tested. In these experiments, the 16S rRNA amplicons were prepared as described in Example 1 using TMA amplification. Labeling reactions of 25 µl and 50 µl were then prepared. The 25 µl-reactions contained: RNA molecules (5µl of TMA reaction mixture), 1.5µl imidazole (0.1 M), 1.5 µl MnCl₂ (1 M), 2 µl 5-(bromomethyl)fluorescein (50 mM in DMF) and 15 µl of pure water. The 50 µl-reactions contained: RNA molecules (5µl of TMA reaction mixture), 3 µl imidazole (0.1 M), 3 µl MnCl₂ (1 M), 2 µl 5-(bromomethyl)fluorescein (50 mM in DMF) and 37 µl of pure water. Both volumes were mixed and incubated at 65°C for 30 min.

After incubation, pure water was added to each mixture to bring the final volume to 100 µl. Then, the labeling reaction products was hybridized, detected and analyzed on a DNA-chip using the manufacturer's protocol (GENECHIP®, Affymetrix, Santa Clara, CA, USA), using the 4-L tiling DNA chip as described above.

The results are shown below.

| Labeling volume | BC% | S | B | S/B |
|---|---|---|---|---|
| 25 µl | 98.4 | 4532 | 2363 | 1.9 |
| 50 µl | 98.4 | 5682 | 2462 | 2.3 |

These results show that labeling reactions can be performed in smaller volumes without affecting base calling or intensity levels.

To increase the S/B ratio, a purification step was included after the fragmentation and labeling reaction was completed. Here, organic solvents in a washing buffer were used to reduce the amount of unattached labeling agent in the mixture that was applied to the DNA chip for detection of the labeled RNA fragments.

Amplicons of 16S rRNA of *M. tuberculosis* were prepared as described in Example 1. Fragmentation and labeling of the amplicons were performed in 25 µl reactions as described above. After fragmentation and labeling, pure water was added to obtain a final volume of 100 µl. Then, the reaction product was hybridized, detected and analyzed on a DNA chip as described above, except that the washing buffers contained either 5% (v/v) of N,N-dimethylformamide (DMF) or 5% (v/v) of dimethyl sulfoxide (DMSO).

These results are shown below.

| Assay | Description | BC% | S | B | S/B |
|---|---|---|---|---|---|
| 1 | Wash buffer without solvent | 96.8 | 13263 | 8420 | 1.6 |
| 2 | Wash buffer with 5% DMF | 98.4 | 5748 | 1399 | 4.1 |
| 3 | Wash buffer with 5% DMSO | 97.3 | 5900 | 1655 | 3.6 |

The results show that the addition of organic solvents in washing buffers reduces the background levels, probably by increasing the solubility of unattached 5-(bromomethyl)fluorescein label in the washing buffer, thereby efficiently removing it from the DNA chip. The base call percentage was also higher in assays that included DMSO or DMF in the washing buffers.

As another method for improving detection on a DNA chip, an extraction of the fragmentation and labeling reaction mixture with an organic solvent was included before the hybridization step. The fragmentation and labeling reaction mixture contained: 16S rRNA amplicons (50 µl of TMA), 15 µl imidazole (0.1 M), 15 µl MnCl₂ (1 M), 5 µl 5-(bromomethyl) fluorescein (50 mM in DMSO) and pure water added for a final volume of 250 µl. The reaction mixture was mixed and incubated at 65°C for 30 min.

Then, 100 µl of the reaction mixture was extracted by using 900 µl of water-saturated 1-butanol. After 1-butanol addition, the solution was vigorously vortex mixed and centrifuged to separate the aqueous and organic phases. 100 µl of the aqueous phase was mixed with 700 µl hybridization buffer (5X SSPE, 3 M betaine, 5 mM DTAB, 250 µg/ml salmon DNA) and hybridization on the DNA chip was performed as described above. Using this procedure, the BC was 98.4%, the signal was 2131 RFU and the background was 314 RFU, resulting in a S/B ratio of 6.8. These results show that the background level was reduced by use of an organic solvent extraction after the fragmentation and labeling reaction, without loss of base call percentage.

To determine if larger volume reactions could similarly be used, an entire 100 µl TMA reaction volume was used. In this case, labeling reagents were added directly in TMA reaction tube as follows. To the TMA reaction (100 µl) were added: 15 µl imidazole (0.1 M), 15 µl MnCl₂ (1 M), 5 µl 5-(bromomethyl)fluorescein (50 mM in DMF) and pure water to a final volume of 250 µl. The reaction medium was mixed and incubated at 65°C for 30 min.

Alternatively, the same protocol was performed, followed by a 1-butanol purification performed substantially as describe above. Then, for both procedures (i.e., with and without organic solvent extraction), 100 µl of the reaction product was hybridized, detected and analyzed on a DNA chip as described above. These results are shown below.

| Description | BC% | S | B | S/B |
|---|---|---|---|---|
| 100 µl volume, without extraction | 97.8 | 1631 | 728 | 2.2 |
| 100 µl volume, with extraction | 98.4 | 3634 | 753 | 4.8 |

This protocol allowed fragmentation and labeling of the amplicons without an additional transfer step of amplicons to another tube because the labeling reagents were added to the TMA tube following amplification.

Similar assays were performed using HIV-1 amplicons produced as described in Example 1. These fragmentation and labeling reactions (250 µl) contained: HIV-1 protease RNA amplicons (50 µl), 15 µl imidazole (0.1 M), 15 µl MnCl₂ (1 M), 5 µl 5-(bromomethyl)fluorescein (100 mM in DMSO) and 165 µl of pure water; and were mixed and incubated at 60°C for 30 min. For organic solvent extractions, two extractions using 1-butanol were performed as described above using 250, 300, 400 or 1000 µl butanol per extraction. Then the reaction product was hybridized, detected and analyzed on a DNA chip as described above, using a DNA chip for detection of the HIV-1 protease gene (Kozal et al., 1996, Nature Med. 2(7): 753-758). The results, shown below, indicate that two organic solvent extractions substantially decreased background for all of the volumes used.

| **Extraction volume** | **BC%** | **S** | **B** | **S/B** |
|---|---|---|---|---|
| 2 x 250 µl | 98.2 | 900 | 274 | 4.3 |
| 2 x 300 µl | 95.3 | 1103 | 214 | 6.2 |
| 2 x 400 µl | 96.6 | 1585 | 271 | 6.8 |
| 2 x 1000 µl | 96.6 | 1003 | 216 | 5.6 |

The protocol was modified by adding EDTA (10 mM) to the fragmentation and labeling reaction mixture before the 1-butanol extraction step. This addition increased solubility of the labeled RNA fragments and prevented precipitation caused by Mn²⁺ metal ions. As shown below, addition of EDTA improved the detection step both for BC and increasing the S/B ratio.

| Description | BC% | S | B | S/B |
|---|---|---|---|---|
| 1-butanol extraction | 96.2 | 1160 | 251 | 4.6 |
| 1-butanol + EDTA extraction | 98.4 | 1303 | 228 | 5.7 |

In addition to 1-butanol, other organic solvents were also tested using similar extraction procedures, with 16S rRNA amplicons as the target nucleic acid for fragmentation and labeling. The results for each solvent are shown below, showing that a variety of organic solvents can be used to effectively remove unattached labeling agent.

| Extraction solvent | BC% | S | B | S/B |
|---|---|---|---|---|
| 1-Butanol | 94.1 | 5387 | 357 | 15.1 |
| 2-Butanol | 97.3 | 3289 | 291 | 11.3 |
| Isopentyl Alcohol | 94.6 | 4505 | 488 | 9.2 |
| Cyclohexanol | 94.6 | 4079 | 248 | 16.4 |
| 1-Pentanol | 93.0 | 4166 | 398 | 10.5 |
| 1-Butanol/Nitromethane | 93.0 | 4799 | 309 | 15.5 |

As an alternative to organic solvent extraction of the unattached label, precipitation of the fragmented and labeled nucleic acid fragments was also tested. In this experiment, HIV-1 amplicons were prepared as described in Example 1. Each reaction included: RNA amplicons (50 µl), 6 µl imidazole (0.1 M), 6 µl MnCl₂ (1 M), 2 µl 5-(bromomethyl)fluorescein (50 mM in DMSO) and pure water to a final volume of 100 µl; the mixed solution was incubated at 65°C for 30 min. For control reactions in which the fragmented RNA was not precipitated, the hybridization and detection steps were performed as described above using the HIV-1 specific DNA chip. For experimental reactions that included a precipitation step, the precipitation step was performed prior to hybridization on the chip. For precipitation, the reaction mixture was mixed with 700 µl of hybridization buffer (5X SSPE, 3 M betaine, 5 mM DTAB, 25 µg/ml salmon sperm DNA) at room temperature by vortexing. The precipitate was pelleted and the supernatant was removed. The pellet was resuspended in 500 µl of the hybridization buffer and hybridization, detection and analysis were performed on the HIV-1 specific DNA chip as described above.

The results are shown below for both procedures.

| Description | BC% | S | B | S/B |
|---|---|---|---|---|
| No Precipitation | 94.6 | 2008 | 1297 | 1.5 |
| Precipitation | 96.6 | 2315 | 483 | 4.8 |

Without precipitation, the signal intensity was high but the S/B ratio was low (1.5); with precipitation, the background decreased and the S/B ratio increased.

### Example 3: Solid Phase Extraction of Unattached Label

In this example, labeled fragments, following fragmentation and labeling of nucleic acids, were physically separated from unattached label using solid phase extraction.

### Silica Beads Solid Support

The solid phase reagent used was magnetic silica beads. The target nucleic acid was 16S rRNA amplicons, prepared as described in Example 1. The reaction included: 50 µl of TMA reaction mixture, 9 µl imidazole (01 M), 9 µl MnCl₂ (1 M), and 3 µl 5-(bromomethyl)fluorescein (100 mM in DMSO) which were mixed and incubated at 60°C for 30 min.

The solid phase extraction was performed using commercially available reagents, the Wizard PureFaction™ Plasmid DNA Purification System Starter Pack (Promega, Madison, WI, USA). In this procedure, the fragmentation and labeling reaction was mixed with 25 µl of MagneSil Paramagnetic Particles and 1 ml of 4/40 Wash solution, agitated vigorously (by vortex), and then the tube was placed on a magnet support to hold the magnetic beads to the side of the tube. The beads were washed using 1 ml of 80% ethanol and the labeled RNA fragments were eluted by using 100 µl of 10 mM Tris-Cl, pH 8.5. The eluate was hybridized, detected and analyzed on a DNA chip as described above. The results of this purification procedure, compared to those obtained using 1-butanol extraction are shown below.

| **Purification** | **BC%** | **S** | **B** | **S/B** |
|---|---|---|---|---|
| 1-butanol extraction | 99.5 | 1639 | 536 | 3.1 |
| Silica beads purification | 98.4 | 875 | 133 | 6.6 |
| Assay 1 | | | | |
| Silica beads purification | 97.8 | 773 | 135 | 5.7 |
| Assay 2 | | | | |

These results show that solid phase extraction using silica beads reduces background and increases the S/B value.

In a separate experiment, a similar silica bead solid phase extraction was performed but the elution was performed using betaine (5 M dissolved in hybridization buffer, 300 µl). Hybridization was then performed on a DNA chip as described above, using hybridization buffer without betaine. When the labeled fragments eluted with betaine were hybridized, the BC was 99.5%, the signal was 2424 RFU, the background was 170 RFU and the S/B was 14.3.

### Silica Membrane Solid Support

In a separate experiment, a silica membrane was used in place of the magnetic silica beads for post-labeling purification. In this experiment, the fragmentation and labeling reaction was performed using 16S rRNA amplicons in a 100 µl volume reaction as described in Example 2, and the post-labeling purification was performed using a silica membrane (QIAQUICK™ Nucleotide Removal kit, Qiagen, Hilden, Germany). For purification, 0, 15 or 75 µl of 0.5 M EDTA and 685 µl of PN Buffer were added to the reaction mixture and the solution was vortexed vigorously. Sample was then processed following the manufacturer's protocol. The eluate was hybridized, detected and analyzed on a DNA chip as described above.

The results of this purification method, compared to those obtained using a 1-butanol extraction, are shown below, showing that the silica membrane purification is comparable to extraction of unattached label using an organic solvent if EDTA was included in the mixture purified by the silica membrane method.

| **Purification** | **EDTA (mM)** | **BC%** | **S** | **B** | **S/B** |
|---|---|---|---|---|---|
| 1-butanol extraction | - | 94.6 | 7390 | 294 | 25.1 |
| QIAQUICK™ purification | 0 | 88.1 | 391 | 225 | 1.7 |
| | 15 | 95.7 | 8498 | 329 | 25.8 |
| | 75 | 98.4 | 6754 | 246 | 27.5 |

Similar silica membrane purification methods were used in a separate experiment but betaine (5 M) elution from the silica membrane was used to recover the labeled RNA fragments, substantially as described above. In this assay, the BC was 94.6%, the signal was 15836 RFU, the background was 502 RFU and the S/B ratio was 31.5.

### QIAVAC™ Column Solid Support

An additional purification method was performed using a PCR purification kit with PN buffer and a vacuum system (QIAVAC™ 6S system) to draw materials through the column (Qiagen, Hilden, Germany). In this assay, 16S rRNA amplicons were fragmented and labeled as described in Example 2, and then 15 µl of 0.5 M EDTA and 685 µl of PN Buffer were added to the reaction mixture, the solution was vortexed vigorously and processed following the manufacturer's protocol. The column was washed with 1 ml of PE buffer and labeled nucleic acid fragments were eluted, hybridized, detected and analyzed on a DNA chip as described above.

The QIAVAC™ purification method produced 100% base call, signal of 15,984 RFU, background of 390 RFU and a S/B ratio of 41. In comparison, similarly cleaved and labeled fragments that were purified using 1-butanol extraction produced 94.6% base call, signal of 7390 RFU, background of 294 RFU and a S/B ratio of 25.1.

### Gel Filtration Solid Supports

Additional solid phase extraction protocols used gel filtration resins in small spin column format (SEPHADEX™, SEPHACRYL™ and BIOGEL™ resins were tested). These resins allow larger molecules (i.e., the labeled nucleic acid fragments) to be selectively eluted, while retaining the smaller molecules (unreacted labeling agent and other mixture components). The nucleic acid used for testing in these assays was 16S rRNA amplicons prepared substantially as described in Example 1, which was then subjected to fragmentation and labeling substantially as described in Example 2. The general procedure was to equilibrate a commercially available spin column containing a gel filtration resin (e.g., a SEPHADEX™ G-50 spin column from Pharmacia) with a 400 µl volume of a buffer (e.g., Tris-azide buffer, pH 7.4) by applying the buffer and then eluting it by centrifugation (1000x g for 1 min), repeating the procedure, and finally wetting the resin with an additional 400 µl of the same buffer. Then a 100 µl reaction mixture was applied to the prepared column and the labeled nucleic acid fragments were eluted by centrifuging the column at 1000x g for 1 min.

To obtain efficient recovery of labeled nucleic acid fragments from a labeling reaction mixture, EDTA (25 mM to 125 mM) was added and mixed with the labeling reaction mixture before it was applied to the gel filtration resin for purification. Optimal recovery and detection (on a DNA chip, assessed by the S/B ratio) was observed when the EDTA concentration was 25 mM or 50 mM; at EDTA concentrations of 75 mM and higher, the background on the detection chip was increased. Typically, 50 mM EDTA was added to the labeling mixture before it was applied to the gel filtration resin.

The efficiency of this purification procedure was demonstrated using this basic procedure to purify labeled fragments obtained from 16S rRNA amplicons as described above. The purified labeled fragments ("purified + EDTA") were then detected after applying them to a *Mycobacterium-*specific GENECHIP® substantially as described in Example 2. For comparison, the same type of labeled fragments obtained directly from a fragmentation and labeling reaction, without gel filtration purification ("unpurified"), was also applied and analyzed using the same DNA chip procedure. Also, for comparison, the assay was done using the same type of labeled fragments that were purified by gel filtration but without adding EDTA ("purified - EDTA") to the labeling mixture before applying it to the column. These DNA chip analysis results are shown below.

| **Sample** | **%BC** | **Signal (mean)** | **Background** | **S/B Ratio** |
|---|---|---|---|---|
| Unpurified | 89.2 | 13396 | 10334 | 1.3 |
| Purified - EDTA | 77.3 | 625 | 483 | 1.24 |
| Purified + EDTA | 95.7 | 3820 | 1185 | 3.03 |

These results show that gel filtration purification, in the presence of 50 mM EDTA, is a quick and effective method of providing labeled nucleic acid fragments that can be readily detected on a DNA probe array.

### Example 4: Effect of 5-(bromomethyl)fluorescein Concentration on Labeling

In this example, differing amounts of labeling agent were used in the fragmentation and labeling reaction to determine if the labeling agent concentration affected the assay. Amplicons of 16S rRNA were prepared as described in Example 1. The reactions each included: RNA (50 µl of TMA), 9 µl imidazole (0.1 M), 9 µl MnCl₂ (1 M), varying amounts of 5-(bromomethyl)fluorescein (50 mM in DMF) to achieve final concentrations of 1 mM (3 µl), 2 mM (6 µl) or 4 mM (12 µl) and pure water to achieve a final volume of 150 µl. The mixture was mixed and incubated at 60°C for 30 min. Then the labeling reaction mixture was purified using 1-butanol extraction as described above and 100 µl of the purified product was hybridized, detected and analyzed on a DNA chip as described in Example 2. The results are reported below, showing that all of the tested concentrations of labeling agent efficiently labeled the fragmented RNA. Higher signal was produced for the higher concentrations of labeling agent, without a significant increase in the background level.

| **[5-(bromomethyl)fluorescein]** | **BC%** | **S** | **B** | **S/B** |
|---|---|---|---|---|
| 1 mM | 96.8 | 2178 | 311 | 7.0 |
| 2 mM | 96.8 | 3511 | 556 | 6.3 |
| 4 mM | 94.6 | 3836 | 421 | 9.1 |

### Example 5: Effect of pH on Fragmentation and Labeling Reactions

In these experiments, the pH of the imidazole reagent was adjusted to 6, 6.8 and 7.0 for use in the fragmentation and labeling reactions which contained: 16S rRNA amplicons (50 µl of TMA reaction), 45 µl of a pH-adjusted mixture of imidazole (20 mM) and MnCl₂ (200 mM), 5 µl 5-(bromomethyl)fluorescein (100 mM in DMSO) and pure water to a final volume of 150 µl. The reactions were mixed and incubated at 60°C for 30 min. Then, hybridization, detection and analysis were performed on a DNA chip as described in Example 2. The results for the different pH conditions of the reactions are shown below. Based on these results, all of the pH conditions tested produced detectably labeled fragments that provided base calling of 98% or more; pH 7.0 appears to be optimal.

| **Reaction pH** | **BC%** | **S** | **B** | **S/B** |
|---|---|---|---|---|
| 6.0 | 98.9 | 2948 | 202 | 14.6 |
| 6.8 | 98.4 | 4052 | 210 | 19.3 |
| 7.0 | 100 | 4668 | 234 | 19.9 |

### Example 6: Influence of Multivalent Metal Cations and Labeling Agents on DNA Labeling

This example shows the relative efficiencies of fluorescein labeling of different single-stranded oligonucleotides, using different labeling agents and multivalent metal cations in the fragmentation and labeling reactions. Labeling of DNA, DNA containing an internal thiophosphate nucleotide and DNA having a 3'-thiophosphate with 5-(bromomethyl)fluorescein and 6-iodoacetamidofluorescein were compared in the presence or absence of different metal cations.

The oligonucleotides used in the fragmentation and labeling experiments were the following:
GCTCGTTGCGGGACTTAACCCAACAT (SEQ ID NO:4);
GCTCGTTGCGGGACTT-s-AACCCAACAT (SEQ ID NO:5) where «-s-» indicates a thiophosphate between nucleotides at positions 16 and 17; and
GCTCGTTGCGGGACTTAACCCAACAT-s (SEQ ID NO:6) where «-s-» indicates a 3' terminal thiophosphate.

The buffer for the reaction was Tris-HCl buffer, pH 8.1, and the different multivalent metal cations were: Zn²⁺, Mn²⁺, Co²⁺, Ni²⁺, Cd²⁺, Pb²⁺, Ce³⁺, Tb³⁺, Yb³⁺, Cr³⁺, In³⁺, Be²⁺, Sn²⁺, Rb⁺, and Cs⁺ (all in solution with Cl⁻ counterions). The negative control containing no multivalent cations was an equivalent volume of pure water. The labeling agents, dissolved in dry N, N-dimethylformamide (DMF), were: 5-(bromomethyl)fluorescein and 6-iodoacetamidofluorescein (Molecular Probes, Inc., Eugene, OR, USA). The DMF-dissolved compounds were generally added to reaction mixtures such that the final concentration of DMF in the mixture was 5% (v/v).

The typical fragmentation and labeling reaction (100 µl) contained (with final concentrations indicated in parentheses for each component): 30 µl of oligonucleotide (6.667 OD/ml; 0.2 OD = 0.00932 mM), 50 µl of 100 mM Tris-HCl buffer (50 mM), 10 µl of 10 mM metal cation (1 mM) or pure H₂O, and 10 µl of 2.5 mM labeling reagent in DMF (0.25 mM). The reaction mixture was mixed by vortexing and incubated at 60°C for 30 min. The labeled products are purified by adding to the labeling reaction mixture 10 µl of 3 M sodium acetate, pH 5.0, then 150 µl water-saturated 1-butanol, and mixing by vortexing. After the phases separated, the aqueous phase (113 µl) was removed to a clean tube to which was added 400 µl ethanol and the solution was vortexed and incubated on dry ice for 15 min. The mixture was centrifuged 15 min at 14,000 rpm, the supernatant removed and the pellet was washed with 100 µl of cold 70% ethanol. The pellet was resuspend in 500 µl of 100 mM sodium carbonate buffer, pH 9.5. The products (labeled DNA and unlabeled DNA) were measured using UV spectroscopy or reverse phase HPLC and peak integration.

The results of these experiments are summarized in Table 1 for labeling of oligonucleotides of SEQ ID NO:5 and SEQ ID NO:6 with 5-(bromomethyl)fluorescein ("5-BMF") and 6-iodoacetamidofluorescein ("6-IA-F") in the presence of 15 multivalent metal cations.

**Table 1: Percent fluorescein incorporation (labeling yield).**

| Metal Cation | **SEQ ID NO:5** | | **SEQ ID NO:6** | |
|---|---|---|---|---|
| | **5-BMF** | **6-IA-F** | **5-BMF** | **6-IA-F** |
| None (H₂O) | 0.933 | 3.10 | 20.8 | 58.6 |
| Yb³⁺ | 78.7 | 48.4 | 98.6 | 60.9 |
| Ce³⁺ | 62.9 | 46.7 | 69.4 | 56.5 |
| Tb³⁺ | 51.8 | 45.4 | 66.9 | 64.2 |
| In³⁺ | 44.8 | 32.7 | 42.8 | 34.1 |
| Pb²⁺ | 43.6 | 54.5 | 59.8 | 69.0 |
| Cr³⁺ | 24.5 | 29.8 | 33.7 | 62.3 |
| Zn²⁺ | 21.1 | 20.5 | 13.6 | 36.0 |
| Be²⁺ | 12.6 | 10.4 | 13.5 | 27.0 |
| Cd²⁺ | 4.3 8 | 4.70 | 5.72 | 22.3 |
| Co²⁺ | 3.94 | 5.52 | 17.2 | 61.2 |
| Sn²⁺ | 3.59 | 4.13 | 23.1 | 50.2 |
| Mn²⁺ | 2.70 | 2.50 | 28.4 | 63.7 |
| Ni²⁺ | 1.70 | 2.22 | 19.5 | 67.0 |
| Cs⁺ | 1.17 | 1.12 | 22.0 | 58.7 |
| Rb⁺ | 1.04 | 1.09 | 20.4 | 57.8 |

Labeling of the DNA containing an internal thiophosphate (SEQ ID NO:5), using either 5-BMF or 6-IA-F, was enhanced, relative to the negative control, by the presence of trivalent metal ions and divalent Pb²⁺, Zn²⁺, and Be²⁺ but not by the other metal cations tested. There is little difference between the two labeling agents when a common metal cation was used. Labeling of the nucleic acid with a 3'-thiophosphate (SEQ ID NO:6) by 5-BMF was enhanced relative to the negative control by the presence of Pb²⁺ and the trivalent metal cations tested, but not by the other cations tested. Labeling of the nucleic acid with a 3'-thiophosphate (SEQ ID NO:6) by 6-IA-F was enhanced relative to the negative control primarily by Pb²⁺ and Ni²⁺. Labeling by 6-IA-F of SEQ ID NO:6 was relatively high in the negative control without metal cations.

The labeling yield obtained with the oligonucleotide that contains no thiophosphate group (SEQ ID NO:4) was below 10% for all of the metal cations tested. The thiophosphate-free DNA was not detectably labeled in the absence of metal cations (i.e., the water control).

### Example 7: Influence of Metal Cations and Labeling Agents on RNA-α-s Labeling

This example shows the relative attachment of fluorescein onto thiophosphate-containing RNA (RNA-α-S), using different labeling agents and metal cations. Fluorescein labeling of RNA containing internal or 3'-terminal thiophosphates was compared using two labeling agents, 6-IA-F and 5-iodoacetamidofluorescein (5-IA-F), in the presence or absence of different metal cations.

The protocol for the experiment is similar to the protocol described in Example 6 but the oligonucleotides used were RNA polymers having the following sequences:
GCUCGUUGCGGGACUU-s-AACCCAACAU (SEQ ID NO::7), where "-s-" indicates a thiophosphate between the two nucleotides at positions 16 and 17; and
GCUCGUUGCGGGACUUAACCCAACAU-s (SEQ ID NO:8), where "-s-" indicates a 3' terminal thiophosphate.

In these labeling reactions the metal cations tested were Mg²⁺, Cr³⁺, Mn²⁺, Ni²⁺, Zn²⁺, In³⁺, Pb²⁺, Ce³⁺, Eu³⁺, Tb³⁺ and Yb³⁺ (all with Cl⁻ counterions); pure water was the negative control. The labeling agents, 5-IA-F and 6-IA-F, were each dissolved in dry DMF. The 100 µl reactions contained (with final concentrations indicated in parentheses): 50 µl of 100 mM Tris-Cl buffer, pH 8.1 (50 mM), 30 µl of 6.667 OD per ml oligomer (0.2 OD = 0.00932 mM final), 10 µl of 10 mM metal cation (1 mM) or pure water, and 10 µl of 2.5 mM labeling reagent (0.25 mM); final DMF concentration was 5%. The combination was mixed by vortexing and incubated at 60°C for 30 min.

After purification using 1-butanol extraction as described in Example 6, a 120 µl of aqueous phase was precipitated with ethanol and the pelleted nucleic acid was resuspended in 500 µl of 100 mM sodium carbonate buffer, pH 9.5. The labeling yield was analyzed by UV spectroscopy as described in Example 6. The results are summarized in Table 2, in which "ND" means not determined. Results for testing with Mn²⁺, Zn²⁺ and In³⁺ are not included in the table because they exhibited relatively low activity.

**Table 2 : Percent fluorescein labeling using different labeling agents, metal cations and oligonucleotides.**

| **Metal Cation** | **SEQ ID NO: 7** | | **SEQ ID NO:8** | |
|---|---|---|---|---|
| | **5-IA-F** | **6-IA-F** | **5-IA-F** | **6-IA-F** |
| **Cr³⁺** | 11.3 | 23.0 | 19.2 | 31.2 |
| **Ce³⁺** | 28.1 | 19.2 | 30.2 | 20.1 |
| **Pb²⁺** | 26.5 | ND | 27.1 | 21.4 |
| **Yb³⁺** | 16.8 | 20.5 | 20.7 | 22.8 |
| **Tb³⁺** | 26.3 | 21.8 | 18.1 | 17.9 |
| **Eu³⁺** | 21.5 | 11.1 | 13.1 | 15.3 |
| **Ni²⁺** | 0 | 0 | 4. 83 | 1.94 |
| **Mg²⁺** | 0 | 0 | 0.774 | 1.98 |
| **H₂O** | 0 | 0.090 | 1.11 | 4.35 |

Labeling of RNA, like DNA, is metal cation and labeling agent sensitive. Both 5-IA-F and 6-IA-F labeled RNA-α-S to similar extents, with the 3'-terminal RNA-α-S being labeled slightly better than the RNA containing an internal thiophosphate. Trivalent metal cations or Pb²⁺ provided the most enhanced labeling relative to the negative control with both labeling agents

### Example 8: Efficiency of RNA Fragmentation by Metal Cations

This example shows the relative efficiencies of RNA fragmentation in the presence of various metal cations. The substrates for fragmentation in these experiments were synthetic RNA oligonucleotides having the following sequences:
GCUCGWGCGGGACUUAACCCAACAU (SEQ ID NO:9), and
GCUCGWGCGGGACUU-s-AACCCAACAU (SEQ ID NO:7), where «-s-» indicates a thiophosphate between nucleotides 16 and 17.

Synthetic complimentary RNA sequences to SEQ ID NO:7 and SEQ ID NO:9 were made and used to form a double-stranded RNA with the appropriate complementary oligonucleotide. For fragmentation testing, both single-stranded and double-stranded RNA were used.

The probe used in the detection step was a 26-nucleotide sequence complimentary to SEQ ID NO:9, with an acridinium ester (AE) label between positions 16 and 17 (described in Nelson et al., 1996, Nucleic Acids Res. 24(24): 4998-5003) The general method involves fragmenting 100 fmoles of RNA in a total volume of 100 µl (i.e., the final RNA concentration in the reaction is 1 fmol/µl) in 50 mM imidazole buffer at pH 7.6, containing 2.5 µmoles of each of the metal cations tested. After the reaction incubated at 60°C for 30 min, the fragmentation reaction was stopped by adding a 2- to 3-fold molar excess of EDTA, pH 8, relative to the metal cation concentration and incubating at -20°C. To monitor the amount of RNA fragmentation, about 5 fmol of RNA was taken from the mixture and probed with a 20-fold excess of the AE-labeled probe. All probe hybridizations were done at 60°C for 60 min (in 1X PACE® 2 hybridization buffer, Gen-Probe, San Diego CA, USA).

Probing a small amount of RNA from the fragmented material also allows the fragmentation mixture volume to be adequately diluted in the hybridization volume such that the fragmentation reaction components do not affect the hybridization of the probe to the target. About 200 µl of PACE® 2 selection reagent (Gen-Probe Incorporated, San Diego CA, USA) was used to hydrolyze the unhybridized probe. The hybridized probe was detected using a luminometer to detect chemiluminescence (as previously described by Nelson et al., *supra*). The chemiluminescence is expressed as relative light units or RLU.

For the double-stranded RNA, about 160 pmol of SEQ ID NO:9 was hybridized with 3-fold excess of the complimentary sequence in 1X PACE® 2 hybridization buffer at 65°C for 30 min. The 3-fold excess complimentary strand concentration is used to ensure complete hybridization. The AE-labeled probe has exactly the same sequence and, therefore, the excess strand will not bind to the probe.

A control reaction without metal cations (i.e., substituting pure water) was done in parallel, and the chemiluminescence signal of the control was used to calculate the percentage of fragmentation.

The metal cations chosen for fragmentation can be broadly divided into three different categories: (1) non-transition metals such as Mg, Sr, Ba (Group II), and Pb (Group IV); (2) transition metals, such as Zn, Cd, Mn, Fe, Co, Ni, and Ru, and (3) lanthanides, such as Ce, Eu, Tb, Tm, Yb, and Lu. The counterion for all the metal cations tested was Cl⁻ (all from Aldrich Chemical Co., Milwaukee, WI, USA).

Table 3 shows the fragmentation efficiency of eighteen different multivalent metal cations at 0.25 mM on single- and double-stranded RNA in 50 mM imidazole buffer (pH 7.6), where the reaction was incubated at 60°C for 30 min. All results are expressed as a percentage of fragmentation.

**Table 3: Percent RNA fragmentation by metal cations.**

| | **Single stranded RNA** | | **Double stranded RNA** | |
|---|---|---|---|---|
| **Metal** | SEQ ID NO:9 | SEQ ID NO:7 | SEQ ID NO:9 | SEQ ID NO:7 |
| **Mg²⁺** | 18 | 62 | 71 | 63 |
| **Sr²⁺** | 26 | 74 | 72 | 63 |
| **Ba²⁺** | 38 | < 5 | 60 | 70 |
| **Pb²⁺** | 82 | 92 | 53 | 55 |
| **Zn²⁺** | 38 | 14 | 11 | 29 |
| **Cd²⁺** | 86 | < 5 | 62 | 60 |
| **Mn²⁺** | 38 | 10 | 72 | 65 |
| **Fe²⁺** | 62 | 7 | < 5 | < 5 |
| **Co²⁺** | 33 | 47 | 72 | 69 |
| **Ni²⁺** | 69 | 58 | 68 | 52 |
| **Ru³⁺** | 95 | 95 | 72 | 60 |
| **Ce³⁺** | 58 | 58 | < 5 | 27 |
| **Eu³⁺** | 52 | 35 | < 5 | 16 |
| **Tb³⁺** | 40 | 52 | < 5 | < 5 |
| **Tm³⁺** | 43 | 57 | < 5 | < 5 |
| **Yb³⁺** | 45 | 54 | < 5 | < 5 |
| **Lu³⁺** | 48 | 52 | < 5 | < 5 |

All the lanthanides fragmented single-stranded RNA more efficiently than double-stranded RNA. Among the transition metals, Zn and Fe fragmented double-stranded RNA less efficiently than single-stranded RNA. Most of the transition and the non-transition metals efficiently fragmented double-stranded RNA.

### Example 9: Efficiency of Fragmentation of Single-stranded RNA by Metal Cations in the Presence of Amplification Buffer

This example shows that fragmentation of single-stranded RNA can also be accomplished in other buffer conditions, such as in the TMA solution conditions. The procedures used are substantially the same as described in Example 8, with the following exceptions.

Negative TMA amplification reactions were performed as described in Example 1 in the absence of target RNA. The negative amplification reactions were pooled together to constitute a TMA solution. The synthetic RNA oligonucleotide (SEQ ID NO:7) was then spiked into this TMA solution for the fragmentation reactions. Four different fragmentation buffers were used, all at pH 7.5: imidazole, MOPS, HEPES, and PIPES (all from Aldrich Chemical Co., Milwaukee, WI, USA). The buffers varied in concentrations from 50 mM to 200 mM with the TMA solution containing the synthetic RNA oligonucleotide diluted to a ratio of 1:20 or not diluted (ratio 1 :1). Three different metal cations were tested (Zn²⁺, Ce³⁺ and Tb³⁺), all at 5 mM.

The fragmentation yield was determined as described in Example 8 and the results summarized in Table 4 for the 1:20 dilution assays. Without dilution of the TMA solution, fragmentation was below 10%.

**Table 4: Percent single-stranded RNA fragmentation by metal cations in amplification buffer.**

| | **Imidazole** | | **MOPS** | | **HEPES** | **PIPES** |
|---|---|---|---|---|---|---|
| **Metal** | 50 mM | 200 mM | 50 mM | 200 mM | 50 mM | 50 mM |
| **Zn²⁺** | 3 | 22 | 20 | 25 | 22 | 22 |
| **Ce³⁺** | 62 | 75 | 68 | 69 | 75 | 79 |
| **Tb³⁺** | 50 | 70 | 57 | 60 | 71 | 61 |

These results show that fragmentation of RNA is also effective in a variety of buffering conditions in the presence of multivalent metal cations, particularly Ce³⁺.

### Example 10: Fragmentation and Labeling of RNA and DNA-α-s As Detected by MALDI-TOF

This example shows fluorescein labeling of oligonucleotide fragments in the presence and absence of metal cations and whether fragmentation is associated with labeling. Fluorescein attachment to the oligonucleotides was quantified by absorption spectroscopy and compared to oligonucleotide fragments detected by MALDI-TOF (matrix-assisted laser desorption/ionization-time-of-flight) mass spectroscopy .

The fragmentation reactions were performed substantially as described in Example 6 using synthetic RNA and DNA oligonucleotides having the following sequences:
GCUCGUUGCGGGACUU (SEQ ID NO: 10),
GCUCGWGCGGGACUU-s (SEQ ID NO:11), which is identical to SEQ ID NO:10 but includes a 3'-terminal thiophosphate,
GCUCGWGCGGGACUU-s-AACCCAACAU (SEQ ID N0:7), and
GCTCGTTGCGGGACT T-s-AACCCAACAT (SEQ ID NO:5), where -s- indicates a thiophosphate between the bases 16 and 17.

In these assays, the buffer was imidazole, and the metal cations were Zn²⁺or Tb³⁺ (both with Cl⁻ counterions); pure water was the negative control. The labeling agents, in dry DMF, were 5-(bromomethyl)fluorescein or 6-iodoacetamidofluorescein. The 100 µl reactions contained (final concentrations shown in parentheses): 50 µl of 100 mM imidazole buffer, pH 7.6 (50 mM), 30 µl of 6.667 OD/ml oligomer (0.2 OD = 0.00932 mM final), 10 µl of 25 mM metal cation (2.5 mM) or pure water, and 10 µl of 2.5 mM labeling reagent (0.25 mM), or pure water for the fragmentation-only reactions.

The reaction mixtures were mixed using vortexing and incubated at 60°C for 30 min. After a purification step, 10 µl of sodium acetate (3 M, pH 5.0) and 150 µl of water-saturated 1-butanol were added and the mixture was mixed by vortexing, then incubated on dry ice for 15 min, centrifuged 15 min at 14,000 rpm, and the pellet was washed with 100 µl of cold 70% ethanol. The pellet was resuspended in 40 µl of pure water, and 3 µl were reserved for MALDI-TOF detection. The remaining portion was diluted to 500 µl with 100 mM sodium carbonate buffer, pH 9.5 for UV-visible spectrophotometry.

For MALDI-TOF detection, a 3 µl sample of 5 OD/ml oligomer (0.015 OD) was mixed with 1 µl of 30 mM ammonium citrate buffer, pH 9.4, and 6 µl of 50 mg/ml 3-hydroxypicolinic acid (HPA, matrix) and a cation exchange resin by triturating about 10× with a pipeting device. The resin was allowed to settle, and a 2 µl sample was spotted onto a gold support, air dried for 20 min, and the mass data were collected using a PerSeptive Biosystems Voyager DE MALDI-TOF mass spectrometer. Masses were based on using the SEQ ID NO:10 oligonucleotide as an external standard.

MALDI-TOF labeling and fragmentation results of the reactions using 5-BMF and 6-IA-F, with or without the cations Zn²⁺ or Tb³⁺, are summarized in Tables 5 and 6. In Table 5, "-" indicates no fluorescein labeled oligomers detected, "+" indicates monofluorescein labeled oligomer detected, and "++" indicates difluorescein labeled oligomer detected. In Table 6, "-" indicates no fragmented products detected, "+" indicates a few fragmented products/groups (2 - 5) detected, "++" indicates more (8 - 12) fragmented products detected, and "+++" indicates many fragmented products detected (> 15). "ND" means not detected.

**Table 5 : Fluorescein Labeling of RNA and DNA oligonucleotides determined by MALDI-TOF mass spectrometry.**

| **Labeling Agent** | **Metal** | **SEQ ID NO:11 (RNA)** | **SEQ ID NO:10 (RNA)** | **SEQ ID NO:7 (RNA)** | **SEQ ID NO:5 (DNA)** |
|---|---|---|---|---|---|
| 5-BMF | Zn²⁺ | + | + | I | + + |
| | Tb³⁺ | - | ND | - | + |
| | None (H₂O) | - | - | - | - |
| 6-lA-F | Zn²⁺ | - | - | + | 11 |
| | Tb³⁺ | - | - | ND | - |
| | None (H₂O) | - | - | - | - |

**Table 6 : Fragmentation of RNA and DNA oligonucleotides determined by MALDI-TOF mass spectrometry.**

| **Labeling Agent** | **Metal** | **SEQ ID NO:11 (RNA)** | **SEQ ID NO:10 (RNA)** | **SEQ ID NO:7 (RNA)** | **SEQ ID NO:5 (DNA)** |
|---|---|---|---|---|---|
| 5-BMF | Zn²⁺ | ++ | +++ | ++ | - |
| | Tb³⁺ | ++ | ND | +++ | ++ |
| | None (H₂O) | - | - | + | - |
| 6-IA-F | Zn²⁺ | - | - | 11 | - |
| | Tb³⁺ | ++ | +++ | ND | ++ |
| | None (H₂O) | - | | 1 | - |
| | Zn²⁺ | | | + | |
| | None (H₂O) | | | + | |

The quantitation of fluorescein labeling, detected by spectrophotometry, is shown in Table 7 as the percent of fluorescein attached to the oligonucleotide in the presence and absence of metal cations.

**Table 7 : Percentage of Fluorescein Attached**

| **Labeling Agent** | **Metal ion** | **SEQ ID NO:11 (RNA)** | **SEQ ID NO:10 (RNA)** | **SEQ ID NO:7 (RNA)** | **SEQ ID NO:5 (RNA)** |
|---|---|---|---|---|---|
| **5-BMF** | Zn²⁺ | 34.0 | 48.4 | 40.6 | 119. |
| | Tb³⁺ | 23.2 | 23.9 | 15.3 | 31.1 |
| | None (H₂O) | 5.21 | 0.511 | 5.05 | 0.683 |
| **6-IA-F** | Zn²⁺ | 21.0 | 27.0 | 31.3 | 125. |
| | Tb³⁺ | 8.17 | 6.32 | 4.82 | 16.0 |
| | None(H₂O) | 3.88 | 0.579 | 0.939 | 1.63 |

These results show that both labeling agents, 5-BMF and 6-IA-F, were effective in labeling RNA oligonucleotide in the presence of Zn²⁺ and Tb³⁺; in the absence of metal cations there was little labeling by either labeling agent. Mass spectroscopy detected singly and doubly fluorescein-labeled oligonucleotide only when the efficiency of labeling was greater than 30% and 40%, respectively. Fluorescein labeling was efficient in the presence of Zn²⁺ on all of the oligomers tested with 5-BMF and on the longer RNA and DNA oligomers with 6-IA-F. In the presence of Zn²⁺ plus either alkylating agent, significantly more fragmentation occured than in the absence of alkylating agent. Tb³⁺ plus alkylating agents fragments DNA.

### Example 11: Fragmentation and Labeling of HIV-1 Amplicons Using CeCl₃ as the Metal Cation Source

This example shows that Ce³⁺ ions can effectively fragment HIV-1 amplicons which are labeled in the same reaction mixture with a fluorescent marker, providing nucleic acid fragments for detection on a DNA chip. The amplicons of HTV-1 protease sequence were prepared as described in Example 1. For fragmentation and labeling the reaction included; 50 µl HIV-1 RNA amplicons, 125 µl imidazole buffer (0.1 M), 12.5 µl of CeCl₃ (100 mM), 6.25 µl of 5-(bromomethyl)fluorescein (10 mM in DMSO), and pure water to obtain a final reaction volume of 250 µl. The solution was mixed and incubated at 60°C for 30 min. Then the reaction product was hybridized, detected and analyzed on an HIV. 1 DNA chip as described in Example 2.

The results show that base calling was 98.1% for a median signal of 1656 RFU, with a background of 390 RFU, providing a S/B ratio of 4.2. Thus, use of CeCl₃ allows effective fragmentation and labeling by 5-(bromomethyl)fluorescein.

### Example 12: Fragmentation and Labeling as a Decontamination Tool

TMA reactions were performed as described in Example 1. Fragmentation and labeling reactions were performed in a plastic tube using 100 µl of TMA amplicons from *M. tuberculosis* target with the conditions described in Example 2 except that an upper layer of inert silicon oil was included in the reaction mixture. Following fragmentation and labeling, the reaction mixture was extracted with 2400 µl of 1-butanol (water saturated), as described in Example 2. Following separation of the aqueous and organic layers, TMA reactions were performed using 5 µl from each layer, and using the same volume containing unlabeled target as a positive control. While the unlabeled target gave the expected amplification product, no amplicons were detected in the TMA reactions that used aliquots of either the aqueous or organic layers. These results show that the fragments produced during the fragmentation and labeling reaction cannot be amplified under these conditions. Thus the fragmentation and labeling process can serve as a decontamination procedure.

## Claims

1. A process of Fragmenting and labeling a synthetic or natural nucleic acid, comprising the steps of:
providing a mixture containing a nucleic acid, a labeling agent containing a detectable label, a chemical catalyst, and at least one multivalent metal cation in a substantially aqueous solution,
wherein the nucleic acid is RNA or RNA comprising at least one thiophosphate nucleotide and the multivalent cation is Sr²⁺, Ra²⁺, Pb²⁺, Cd²⁺, Fe²⁺, Ni²⁺, Ru³⁺, Cr³⁺, Ce³⁺, Eu³⁺, Tb³⁺, Tm³⁺, Yb³⁺ or Lu³⁺, and
wherein the nucleic acid is DNA or DNA comprising at least one thiophosphate nucleotide and the multivalent cation is Mn²⁺, Zn²⁺, Be²⁺, Cr³⁺, Pb²⁺, In³⁺, Tb³⁺, Cc³⁺, Yb³⁺ or Ni²⁺ ;
chemically fragmenting the nucleic acid by using the chemical catalyst in the mixture to produce a multiplicity of nucleic acid fragments; and
attaching at least one label to at least one of the nucleic acid fragments to produce a detectably labeled nucleic acid fragment.

2. The process according to claim 1, wherein reagents used in the fragmenting and attaching steps are added to an *in vitro* nucleic acid amplification mixture.

3. The process according to claim 1, further comprising the step of treating the mixture after the fragmenting and attaching steps to decrease or eliminate unattached labeling agent.

4. The process according to claim 3, wherein the treating step consists in adding a quencher to the mixture after the fragmenting and attaching steps.

5. The process according to claim 4, wherein the quencher is a pyrophosphate, thiol derivative, chelating agent, phosphate anion or carbonate anion.

6. The process according to claim 3, wherein the treating step physically separates the labeled nucleic acid fragment from unattached labeling agent in the mixture after the fragmenting and attaching steps.

7. The process according to claim 6, wherein the treating step further includes adding an acid to the mixture after the fragmenting and attaching steps.

8. The process according to claim 6 or 7, wherein the treating step further includes adding a chelating agent to the mixture after the fragmenting and attaching steps.

9. The process according to any one of claims 6 to 8, wherein the treating step uses an organic solvent to separate the labeled nucleic acid fragment from the unattached labeling agent.

10. The process according to claim 9, wherein the organic solvent is 1-butanol, 2-butanol, isopentyl alcohol, 1-pentanol or cyclohexanol.

11. The process according to any one of claims 6 to 8, wherein the treating step separates the labeled nucleic acid fragment from the unattached labeling agent by using solid phase extraction of the nucleic acid fragments on a solid support.

12. The process according to claim 11, wherein said solid support is beads, gels, ion exchange resin, reverse phase resin, silica matrix or a membrane.

13. The process according to claim 11 or 12, wherein the labeled nucleic acid fragment is eluted from the solid support by using a buffer containing betaine.

14. The process according to any one of claims 6 to 8, wherein the treating step precipitates the labeled nucleic acid fragments at ambient temperature from a solution that contains betaine, DTAB and unlabeled nucleic acid.

15. The process according to any one of claims 6 to 8, wherein the treating step dilutes an *in vitro* nucleic acid amplification mixture.

16. The process according to any one of claims 1 to 15, wherein the fragmenting and attaching steps are performed in a single reaction mixture.

17. The process according to any one of claims 1 to 15, wherein the fragmenting and attaching steps are effected in separate steps.

18. The process according to any one of claims 1 to 17, wherein the attaching step attaches a label to an internal or terminal thiophosphate or to an internal or terminal phosphate.

19. The process according to claim 1, wherein the chemical catalyst is a general base selected from the group consisting of imidazole, a substituted analogue of imidazole, and a compound that includes an imidazole ring or substituted analogue of an imidazole ring.

20. The process according to claim 19, wherein the chemical catalyst is selected from the group consisting of N-methylimidazole, MOPS, HEPES, PIPES, and bioorganic polyamines.

21. The process according to any one of claims 1 to 20, wherein the nucleic acid is a DNA and the multivalent metal cation is Tb³⁺.

22. The process according to claim 1, wherein the mixture contains the labeling agent in a concentration of between 0.1 mM to 4 mM.

23. The process according to claim 22, wherein the mixture contains the labeling agent in a concentration of between 0.1 mM to 1 mM.

24. The process according to claim 22 or 23, wherein the labeling agent is between 0.3 mM to 0.55 mM.

25. The process according to claim 1, wherein the mixture contains a labeling agent that contains alkyl halide or haloacetamide reactive functions.

26. The process according to any one of claims 1 to 25, wherein the labeling agent is 5-(bromomethyl)fluorescein, 6-(bromomethyl)fluorescein, 6-iodoacetamidofluorescein or 5-iodoacetamidofluorescein.

## Patentansprüche

1. Verfahren zur Fragmentierung und Markierung einer synthetischen oder natürlichen Nukleinsäure, bei dem man in den folgenden Schritten
ein eine Nukleinsäure, ein Markierungsmittel mit einer nachweisbaren Markierung, einen chemischen Katalysator und wenigstens ein mehrwertiges Metallkation in einer weitgehend wäßrigen Lösung enthaltendes Gemisch bereitstellt,
wobei es sich bei der Nukleinsäure um RNA oder wenigstens ein Thiophosphatnukleotid umfassende RNA und bei dem mehrwertigen Kation um Sr²⁺, Ba²⁺, Pb²⁺, Cd²⁺, Fe²⁺, Ni²⁺, Ru³⁺, Cr³⁺, Ce³⁺, Eu³⁺, Tb³⁺, Tm³⁺, Yb³⁺ oder Lu³⁺ handelt und
wobei es sich bei der Nukleinsäure um DNA oder wenigstens ein Thiophosphatnukleotid umfassende DNA und bei dem mehrwertigen Kation um Mn²⁺, Zn²⁺, Be²⁺, Cr³⁺, Pb²⁺, In³⁺, Tb³⁺, Ce³⁺, Yb³⁺ oder Ni²⁺ handelt, die Nukleinsäure unter Verwendung des chemischen Katalysators in dem Gemisch chemisch fragmentiert, so daß man eine Vielzahl an Nukleinsäurefragmenten erhält, und
wenigstens eine Markierung an wenigstens eines der Nukleinsäurefragmente unter Erhalt eines nachweisbar markierten Nukleinsäurefragments bindet.

2. Verfahren nach Anspruch 1, wobei im Fragmentierungs- und im Bindungsschritt verwendete Reagentien zu einem In-vitro-Nukleinsäureamplifikationsgemisch gegeben werden.

3. Verfahren nach Anspruch 1, bei dem man in einem weiteren Schritt das Gemisch nach dem Fragmentierungs- und dem Bindungsschritt zur Verringerung oder Beseitigung von nicht gebundenem Markierungsmittel behandelt.

4. Verfahren nach Anspruch 3, wobei der Behandlungsschritt aus der Zugabe eines Quenchers zu dem Gemisch nach dem Fragmentierungs- und dem Bindungsschritt besteht.

5. Verfahren nach Anspruch 4, wobei es sich bei dem Quencher um ein Pyrophosphat, ein Thiolderivat, einen Komplexbildner, ein Phosphatanion oder ein Carbonatanion handelt.

6. Verfahren nach Anspruch 3, wobei im Behandlungsschritt das markierte Nukleinsäurefragment von nicht gebundenem Markierungsmittel im Gemisch nach dem Fragmentierungs- und dem Bindungsschritt physikalisch getrennt wird.

7. Verfahren nach Anspruch 6, wobei der Behandlungsschritt ferner die Zugabe einer Säure zu dem Gemisch nach dem Fragmentierungs- und dem Bindungsschritt beinhaltet.

8. Verfahren nach Anspruch 6 oder 7, wobei der Behandlungsschritt ferner die Zugabe eines Komplexbildners zu dem Gemisch nach dem Fragmentierungs- und dem Bindungsschritt beinhaltet.

9. Verfahren nach einem der Ansprüche 6 bis 8, wobei im Behandlungsschritt ein organisches Lösungsmittel zur Trennung des markierten Nukleinsäurefragments vom nicht gebundenen Markierungsmittel eingesetzt wird.

10. Verfahren nach Anspruch 9, wobei es sich bei dem organischen Lösungsmittel um 1-Butanol, 2-Butanol, Isopentylalkohol, 1-Pentanol oder Cyclohexanol handelt.

11. Verfahren nach einem der Ansprüche 6 bis 8, wobei im Behandlungsschritt das markierte Nukleinsäurefragment vom nicht gebundenen Markierungsmittel mittels Festphasenextraktion der Nukleinsäurefragmente an einem festen Träger getrennt wird.

12. Verfahren nach Anspruch 11, wobei es sich bei dem festen Träger um Kügelchen, Gele, Ionenaustauschharz, Umkehrphasenharz, Siliciumdioxidmatrix oder eine Membran handelt.

13. Verfahren nach Anspruch 11 oder 12, wobei man das markierte Nukleinsäurefragment vom festen Träger unter Verwendung eines betainhaltigen Puffers eluiert.

14. Verfahren nach einem der Ansprüche 6 bis 8, wobei im Behandlungsschritt die markierten Nukleinsäurefragmente bei Umgebungstemperatur aus einer Betain, DTAB und nicht markierte Nukleinsäure enthaltenden Lösung ausgefällt werden.

15. Verfahren nach einem der Ansprüche 6 bis 8, wobei ein In-vitro-Nukleinsäureamplifikationsgemisch durch den Behandlungsschritt verdünnt wird.

16. Verfahren nach einem der Ansprüche 1 bis 15, wobei man den Fragmentierungs- und den Bindungsschritt in einem einzigen Reaktionsansatz durchführt.

17. Verfahren nach einem der Ansprüche 1 bis 15, wobei man den Fragmentierungs- und den Bindungsschritt in getrennten Schritten ausführt.

18. Verfahren nach einem der Ansprüche 1 bis 17, wobei im Bindungsschritt eine Markierung an ein innen liegendes oder endständiges Thiophosphat oder an ein innen liegendes oder endständiges Phosphat gebunden wird.

19. Verfahren nach Anspruch 1, wobei es sich bei dem chemischen Katalysator um eine aus der aus Imidazol, einem substituierten Imidazolanalog und einer einen Imidazolring oder ein substiutiertes Analog eines Imidazolrings enthaltenden Verbindung bestehenden Gruppe ausgewählte allgemeine Base handelt.

20. Verfahren nach Anspruch 19, wobei der chemische Katalysator aus der Gruppe N-Methylimidazol, MOPS, HEPES, PIPES und bioorganische Polyamine ausgewählt ist.

21. Verfahren nach einem der Ansprüche 1 bis 20, wobei es sich bei der Nukleinsäure um DNA und bei dem mehrwertigen Metallkation um Tb³⁺ handelt.

22. Verfahren nach Anspruch 1, wobei das Gemisch das Markierungsmittel in einer Konzentration zwischen 0,1 mM und 4 mM enthält.

23. Verfahren nach Anspruch 22, wobei das Gemisch das Markierungsmittel in einer Konzentration zwischen 0,1 mM und 1 mM enthält.

24. Verfahren nach Anspruch 22 oder 23, wobei zwischen 0,3 mM und 0,55 mM Markierungsmittel vorliegen.

25. Verfahren nach Anspruch 1, wobei das Gemisch ein Markierungsmittel enthält, das reaktive Alkylhalogenid- oder Halogenacetamidfunktionen enthält.

26. Verfahren nach einem der Ansprüche 1 bis 25, wobei es sich bei dem Markierungsmittel um 5-(Brommethyl)fluorescein, 6-(Brommethyl)fluorescein, 6-Iodacetamidofluorescein oder 5-Iodacetamidofluorescein handelt.

## Revendications

1. Procédé de fragmentation et de marquage d'un acide nucléique de synthèse ou naturel, comprenant les étapes suivantes :
On dispose d'un mélange contenant un acide nucléique, un agent de marquage contenant un marqueur détectable, un catalyseur chimique, et au moins un cation métallique multivalent dans une solution essentiellement aqueuse,
Etape dans laquelle l'acide nucléique est un ARN ou un ARN qui comprend au moins un nucléotide thiophosphate et le cation multivalent est Sr²⁺, Ba²⁺, Pb²⁺, Cd²⁺, Fe²⁺, Ni²⁺, Ru³⁺, Cr³⁺, Ce³⁺, Eu³⁺, Tb³⁺, Tm³⁺, Yb³⁺ ou Lu³⁺,
Et dans laquelle l'acide nucléique est un ADN ou un ADN qui comprend au moins un nucléotide thiophosphate et le cation multivalent est Mn²⁺, Zn²⁺, Be²⁺, Cr³⁺, Pb²⁺, In³⁺, Tb³⁺, Ce³⁺, Yb³⁺ ou Ni²⁺
On fragmente chimiquement l'acide nucléique en utilisant le catalyseur chimique du mélange pour obtenir une multiplicité de fragments d'acide nucléique, et
On fixe au moins un marqueur sur au moins l'un desdits fragments d'acide nucléique pour obtenir un fragment d'acide nucléique marqué détectable.

2. Procédé selon la revendication 1, **caractérisé en ce que** les réactifs utilisés dans les étapes de fragmentation et de marquage sont ajoutés dans un mélange d'amplification d'acide nucléique *in vitro.*

3. Procédé selon la revendication 1, **caractérisé en ce qu'**il comprend en outre une étape de traitement du mélange après les étapes de fragmentation et de marquage pour diminuer ou éliminer l'agent de marquage non fixé.

4. Procédé selon la revendication 3, **caractérisé en ce que** l'étape de traitement consiste à additionner un désactivateur au mélange après les étapes de fragmentation et de fixation.

5. Procédé selon la revendication 4, **caractérisé en ce que** le désactivateur est un pyrophosphate, un dérivé thiol, un agent chélatant, un anion phosphate ou un anion carbonate.

6. Procédé selon la revendication 3, **caractérisé en ce que**, dans l'étape de traitement, on sépare physiquement le fragment d'acide nucléique marqué de l'agent de marquage non fixé dans le mélange après les étapes de fragmentation et de fixation.

7. Procédé selon la revendication 6, **caractérisé en ce que** l'étape de traitement comprend en outre l'addition d'un acide au mélange après les étapes de fragmentation et de fixation.

8. Procédé selon la revendication 6 ou 7, **caractérisé en ce que** l'étape de traitement comprend en outre l'addition d'un agent chélatant au mélange après les étapes de fragmentation et de fixation.

9. Procédé selon l'une quelconque des revendications 6 à 8, **caractérisé en ce que**, dans l'étape de traitement, on utilise un solvant organique pour séparer le fragment d'acide nucléique marqué de l'agent de marquage non fixé.

10. Procédé selon la revendication 9, **caractérisé en ce que** le solvant organique est le butanol-1, le butanol-2, l'alcool isopentylique, le pentanol-1 ou le cyclohexanol.

11. Procédé selon l'une quelconque des revendications 6 à 8, **caractérisé en ce que**, dans l'étape de traitement, on sépare le fragment d'acide nucléique marqué de l'agent de marquage non fixé, en utilisant une phase solide d'extraction des fragments d'acide nucléique sur un support solide.

12. Procédé selon la revendication 11, **caractérisé en ce que** le support solide est choisi parmi des billes, des gels, une résine échangeuse d'ions, une résine à phase inverse, une matrice de silice ou une membrane.

13. Procédé selon la revendication 11 ou 12, **caractérisé en ce que** le fragment d'acide nucléique marqué est élué du support solide avec un tampon contenant une bétaïne.

14. Procédé selon l'une quelconque des revendications 6 à 8, **caractérisé en ce que**, dans l'étape de traitement, on précipite les fragments d'acide nucléique marqués à température ambiante depuis une solution contenant la bétaïne, DTAB et l'acide nucléique non marqué.

15. Procédé selon l'une quelconque des revendications 6 à 8, **caractérisé en ce que**, dans l'étape de traitement, on dilue un mélange d'amplification d'acide nucléique *in vitro.*

16. Procédé selon l'une quelconque des revendications 1 à 15, **caractérisé en ce que** les étapes de fragmentation et de fixation sont réalisées dans un seul mélange réactionnel.

17. Procédé selon l'une quelconque des revendications 1 à 15, **caractérisé en ce que** les étapes de fragmentation et de fixation sont réalisées séparément.

18. Procédé selon l'une quelconque des revendications 1 à 17, **caractérisé en ce que**, dans l'étape de fixation, on fixe un marqueur sur un thiophosphate interne ou terminal ou sur un phosphate interne ou terminal.

19. Procédé selon la revendication 1, **caractérisé en ce que** le catalyseur chimique est base générale choisie parmi l'imidazole, un analogue substitué de l'imidazole et un composé qui comporte un cycle imidazole ou un analogue substitué d'un cycle imidazole.

20. Procédé selon la revendication 19, **caractérisé en ce que** le catalyseur chimique est choisi parmi le N-méthylimidazole, le MOPS, le HEPES, le PIPES et les polyamines bioorganiques.

21. Procédé selon l'une quelconque des revendications 1 à 20, **caractérisé en ce que** l'acide nucléique est un ADN et le cation métallique multivalent est Tb³⁺.

22. Procédé selon la revendication 1, **caractérisé en ce que** la concentration de l'agent de marquage dans le mélange est comprise entre 0,1 mM to 4 mM.

23. Procédé selon la revendication 22, **caractérisé en ce que** la concentration de l'agent de marquage dans le mélange est comprise entre 0,1 mM to 1 mM.

24. Procédé selon la revendication 22 ou 23, **caractérisé en ce que** la concentration de l'agent de marquage dans le mélange est comprise entre 0,3 mM to 0,55 mM.

25. Procédé selon la revendication 1, **caractérisé en ce que** l'agent de marquage du mélange contient un halogénure d'alkyle ou des fonctions haloacétamide réactives.

26. Procédé selon l'une quelconque des revendications 1 à 25, **caractérisé en ce que** l'agent de marquage est la 5-(bromométhyl)fluorescéine, la 6-(bromométhyl)fluorescéine, la 6-iodoacétamidofluorescéine ou la 5-iodoacétamidofluorescéine.


### SEQUENCE LISTING

<110> BIO MERIEUX
   GEN-PROBE INCORPORATED
<120> PROCESS FOR LABELING A NUCLEIC ACID
<130> CLIVPUR
<140>
   <141>
<160> 11
<170> PatentIn Ver. 2.1
<210> 1
   <211> 43
   <212> DNA
   <213> HIV
<400> 1
   aattaaccct cactaaaggg agacagagcc aacagcccca cca 43
<210> 2
   <211> 54
   <212> DNA
   <213> HIV
<400> 2
   taatacgact cactataggg agatttcccc actaacttct gtatgtcatt gaca 54
<210> 3
   <211> 49
   <212> DNA
   <213> HIV
<400> 3
   taatacgact cactataggg agagggccat ccattcctgg ctttaattt 49
<210> 4
   <211> 26
   <212> DNA
   <213> Artificial Sequence
<220>
   <223> Description of Artificial Sequence:primer
<400> 4
   gctcgttgcg ggacttaacc caacat 26
<210> 5
   <211> 26
   <212> DNA
   <213> Artificial Sequence
<220>
   <223> Description of Artificial Sequence:primer ; the phosphate between nucleotides at positions 16 and 17 is a thiophosphate
<400> 5
   gctcgttgcg ggacttaacc caacat 26
<210> 6
   <211> 26
   <212> DNA
   <213> Artificial Sequence
<220>
   <223> Description of Artificial Sequence:primer ; the phosphate at the 3' end is a terminal thiophosphate
<400> 6
   gctcgttgcg ggacttaacc caacat 26
<210> 7
   <211> 26
   <212> RNA
   <213> Artificial Sequence
<220>
   <223> Description of Artificial Sequence:primer ; the phosphate between the two nucleotides at position 16 and 17 is a thiophosphate
<400> 7
   gcucguugcg ggacuuaacc caacau 26
<210> 8
   <211> 26
   <212> RNA
   <213> Artificial Sequence
<220>
   <223> Description of Artificial Sequence:primer ; the phosphate at the 3' end is a terminal thiophosphate
<400> 8
   gcucguugcg ggacuuaacc caacau 26
<210> 9
   <211> 26
   <212> RNA
   <213> Artificial Sequence
<220>
   <223> Description of Artificial Sequence:primer
<400> 9
   gcucguugcg ggacuuaacc caacau 26
<210> 10
   <211> 16
   <212> RNA
   <213> Artificial Sequence
<220>
   <223> Description of Artificial Sequence:primer
<400> 10
   gcucguugcg ggacuu 16
<210> 11
   <211> 16
   <212> RNA
   <213> Artificial Sequence
<220>
   <223> Description of Artificial Sequence:primer ; the phosphate at the 3' end is a terminal thiophosphate
<400> 11
   gcucguugcg ggacuu 16